Europäisches Patentamt

(19) European Patent Office

Office européen des brevets

(11) Numéro de publication: **0 267 110**
**A1**

## (12) DEMANDE DE BREVET EUROPEEN

(21) Numéro de dépôt: 87402458.1

(22) Date de dépôt: 02.11.87

(51) Int. Cl.⁴: **C 12 N 15/00**
C 12 N 1/20
// C12P21/02, (C12N1/20,
C12R1:19)

(30) Priorité: 03.11.86 FR 8615263

(43) Date de publication de la demande:
**11.05.88 Bulletin 88/19**

(84) Etats contractants désignés:
**AT BE CH DE ES GB GR IT LI LU NL SE**

(71) Demandeur: **INSTITUT MERIEUX Société anonyme dite:**
**17, rue Bourgelat**
**F-69002 LYON (FR)**

(72) Inventeur: **Blanc-Magnoloux, Brigitte**
**24 rue Marcellin Champagnat**
**St Etienne (FR)**

**Heyde, Martine**
**Résidence La Roseraie 6 allée Marcel Achard**
**F-69100 Villeurbanne (FR)**

**Portalier, Raymond Charles Marcel**
**8 avenue Jean Jaurès**
**F-69007 Lyon (FR)**

(74) Mandataire: **Lemoine, Michel et al**
**Cabinet Michel Lemoine et Bernasconi 13 Boulevard des Batignolles**
**F-75008 Paris (FR)**

Le (Les) microorganisme(s) a (ont) été déposé(s) auprès Collection Nationale de Cultures de Microorganismes sous le(s) numéro(s) I-601, I-602, I-603, I-604, I-605.

(54) **Nouvelles souches d'Escherichia coli hyperproductrices et/ou hyperexcrétrices de protéines exportées, procédé d'obtention de ces souches, et application à la production de protéines.**

(57) Nouvelles souches d'Escherichia coli hyperproductrices ou simultanément hyperproductrices et hyperexportatrices de protéines exportées, caractérisées en ce qu'elles comportent une mutation, dite hyp, localisée dans la région MAIA mtl vers la minute 81, ladite mutation étant cotransductible à une fréquence d'environ 20 à 25% par le bactériophage P1 avec le marqueur mtl, ladite mutation étant responsable d'une hyperproduction (et/ou d'une hyperexportation) d'au moins deux protéines d'origine périplasmique présentes, mais en quantité au moins 2 à 5 fois plus faible, dans la souche parentale; ces souches permettent la production accrue de protéines.

EP 0 267 110 A1

## Description

Nouvelles souches d'Escherichia coli hyperproductrices et/ou hyperexcrétrices de protéines exportées, procédé d'obtention de ces souches, et application à la production de protéines.

La présente invention a trait à de nouvelles souches d'Escherichia coli hyperproductrices et/ou hyperexcrétrices de protéines exportées ainsi qu'à un procédé d'obtention de ces souches. L'invention a également trait à l'application de ces souches à la production de protéines, notamment d'enzymes.

La bactérie E. coli possède trois compartiments extracytoplasmiques, à savoir la membrane interne de l'enveloppe cellulaire, l'espace périplasmique et la membrane externe de l'enveloppe cellulaire.

Les protéines cellulaires sont, pour l'essentiel, synthétisées dans le cytoplasme chez la souche sauvage d'E. coli. Le plus grand nombre de ces protéines, les protéines cytoplasmiques, vont y demeurer alors que les autres seront exportées vers les divers autres compartiments cellulaires extracytoplasmiques.

Les mécanismes moléculaires qui assurent l'exportation des protéines à travers les membranes biologiques ne sont que partiellement élucidés. L'un des modèles retenus est le modèle dit de "l'hypothèse du signal". Voir Blobel G., Dobberstein B. (1975), J. Cell. Biol. 67 (835-851 et 851-862). Chez les bactéries, le modèle de l'hypothèse du signal a pu être éprouvé et précisé par une approche génétique. Différentes classes de mutants d'E. coli altérés au niveau de l'exportation ont été récemment isolées. Certaines mutations ont été localisées dans la séquence signal de protéines périplasmiques ou membranaires. Leur étude a permis de montrer que le rôle de la séquence signal est essentiel pour l'initiation du transfert mais que cette séquence ne contient pas toute l'information nécessaire à la translocation. D'autres mutations exercent un effet pléiotrope et pourraient être situées dans les gènes codant pour des constituants jouant un rôle analogue à ceux du complexe SRP ou de la protéine DP. L'exportation de protéines de l'enveloppe bactérienne pourrait également comporter des étapes post-traductionnelles.

On a récemment isolé deux types de mutants de compartimentation, à savoir :

- des mutants excréteurs, qui contrairement à la souche sauvage libèrent spontanément, dans le milieu extracellulaire, certaines enzymes périplasmiques (phosphatase alcaline, β-lactamase) de manière pléiotrope (voir brevet FR-A-8320022) ou spécifique (voir brevet FR-A-8500087)

- des mutants pléiotropes négatifs qui sont déficients en plusieurs protéines périplasmiques et membranaires.

On connaît également des mutants présentant une concentration accrue d'un petit nombre de protéines exportées mais, dans tous ces cas, le mécanisme altéré affecte la régulation spécifique de la synthèse de ces protéines (protéines du régulon maltose : voir Hofnung M. (1982) Ann. Microbiol. 135 5-9 ; porines OmpF et OmpC du régulon omp : voir Hall MN. Silhavy T.J. (1981) J. Mol. Biol. 151 1-15)

Par ailleurs, on a déjà isolé des mutants présentant une exaltation de protéines responsables de l'inactivation d'antibiotiques. Voir Normark S., Edlund T., Grundström T., Bergström S., Wolf Watz M. (1977) J. Bacteriol. 132 912-922 pour la β-lactamase chromosomique, et George A.M., Levy S.B., (1983) J. Bactériol. 155 541-548 pour les protéines permettant l'efflux de la tétracycline. Ces travaux n'ont cependant permis que d'obtenir des mutants exaltant des protéines impliquées spécifiquement dans des mécanismes de résistance à une classe d'antibiotiques.

La présente invention se propose de fournir de nouveaux mutants d'Escherichia coli, notamment K-12, hyperproducteurs et/ou hyperexportateurs de protéines exportées et qui permettent de produire et/ou de libérer dans le compartiment extra-cellulaire, des protéines produites dans le cytoplasme.

Un autre objectif de l'invention est de réaliser de tels mutants se caractérisant par un accroissement élevé de la production de protéines.

L'invention a pour objet de nouvelles souches d'Escherichia coli hyperproductrices et/ou hyperexcrétrices de protéines exportées, caractérisées en ce qu'elles comportent une mutation, dite hyp, localisée dans la région mAlA mtl vers la minute 81, ladite mutation étant cotransductible à une fréquence d'environ 20 à 25 % par le bactériophage P1 avec le marqueur mtl et n'étant pas cotransductible avec les marqueurs ilvA et xyl, ladite mutation étant responsable d'une hyperproduction et/ou d'une hyperexportation d'au moins deux protéines périplasmiques présentes, mais en quantité au moins 2 à 5 fois plus faible, dans la souche parentale.

L'invention a également pour objet les souches de mutants d'E. coli déposées auprès de la Collection Nationale de Culture de Micro-organismes (CNCM) tenue par l'Institut Pasteur et accessibles sous les numéros indiqués ci-dessous :

N° CNCM        Référence de la souche

I-601        3693

I-602        3190

I-603        3181

I-604        3042

Parmi les nouvelles souches d'E. coli selon l'invention figurent, notamment, les souches dont la mutation est responsable d'une hyper-production de la phosphatase alcaline et/ou de la β-lactamase et/ou de l'aminoglycoside 3' phospho-transférase (APH 3').

Dans une forme de réalisation préférée de l'invention, les mutants selon l'invention comportent également les mutations permettant la libération spontanée dans le milieu extra-cellulaire de protéines périplasmiques de manière pléiotrope, telles que décrites dans le brevet FR-A-8320022 ou, de préférence, spécifique telles que décrites dans le brevet FR-A-8500087.

De façon préférée, la bactérie selon l'invention comporte aussi la mutation d'excrétion spécifique exc876, de préférence associée à la présence d'un plasmide exaltant la synthèse de la protéine pertinente.

L'invention a également pour objet un procédé d'obtention des souches selon l'invention, caractérisé en ce que l'on choisit ou construit une souche parentale d'Escherichia coli synthétisant, à un niveau faible mais décelable, la ou les protéines dont on désire obtenir l'exaltation de la production et/ou de l'excrétion, ainsi qu'au moins une enzyme susceptible de détruire un agent anti-bactérien, tel que, de préférence, un antiboitique, et présente dans le milieu cellulaire à une concentration au moins décelable, certaines au moins de ces protéines, ou enzymes n'appartenant pas au même régulon, que l'on cultive la souche parentale et que l'on sélectionne la ou les bactéries présentant une synthèse accrue de ladite enzyme et une résistance accrue auxdits agents anti-bactériens, le taux d'obtention des mutants étant de l'ordre de $10^{-8}$.

L'invention a également pour objet l'application de ces nouvelles souches à la production de protéines, et notamment d'enzymes telles que la phosphatase alcaline, la β-lactamase et/ou l'amino-glycoside 3' phospho-transférase par culture d'une souche dans un milieu liquide approprié, et recueil du liquide de culture séparé des bactéries, de préférence après une durée de culture de l'ordre de 18 heures.

D'autres avantages et caractéristiques de l'invention apparaîtront à la lecture de la description suivante faite à titre d'exemple non limitatif.

1. Souches bactériennes parentales.

Toutes les souches utilisées dérivent d'Escherichia coli K-12.

La souche parentale est construite de sorte que les enzymes choisies soient synthétisées à des taux relativement faibles mais aisément décelables par dosages quantitatifs sur des extraits bruts ou par test qualitatif au niveau des colonies, afin de faciliter l'identification des mutants définis par un contenu accru en ces enzymes.

Pour la β-lactamase chromosomique (gène de structure ampC), on a introduit dans la souche parentale la mutation ampA1. En effet, cette mutation de type "promoteur up" du gène ampC permet de rendre le dosage de l'enzyme plus précis et confère aux souches qui la portent un niveau de résistance reproductible à l'ampicilline = à 10 μg/ml.

Pour L'APH3', dont le gène de structure est porté par le transposon Tn5, une insertion gal::Tn5 a été introduite par transduction dans le génome de la souche parentale : ce génotype confère une résistance reproductible à 50μg/ml de kanamycine

Pour la phosphatase alcaline, le taux de synthèse a été fixé à une valeur faible pour qu'une exaltation même limitée soit détectable au niveau des colonies par le test coloré défini ci-dessous. La combinaison des mutations phoR51 et phoU a été retenue : elle correspond à une faible synthèse constitutive après croissance des bactéries sur milieu riche.

Les propriétés caractéristiques des souches utilisées et construites sont décrites dans le tableau suivant :

| Souches | Sexe | génotypes |
|---|---|---|
| N5230 | Hfr | proC gal::Tn5 met |
| 2590 | Hfr | met gal::Tn5 ampA1 phoR51 phoU zib::Tn10 |
| 3031 | Hfr | met gal::Tn5 ampA1 phoR51 phoU zib::Tn10 hyp3031 |
| 3034 | Hfr | met gal::Tn5 ampA1 phoR51 phoU zib::Tn10 hyp3034 |
| 3039 | Hfr | met gal::Tn5 ampA1 phoR51 phoU zib::Tn10 hyp3039 |
| 3041 | Hfr | met gal::Tn5 ampA1 phoR51 phoU zib::Tn10 hyp3041 |
| 3042 | Hfr | met gal::Tn5 ampA1 phoR51 phoU zib::Tn10 hyp3042 |
| hyp... | Hfr | met gal::Tn5 ampA1 phoR51 phoU zib::Tn10 hyp... |
| CSH 57A | F⁻ | ara leu lacY proA proC purE trp his argH ilv metA ou B gal malA strA xyl mtl |
| 2902 | F⁻ | ara leu lacY proA proC purE trp his argH ilv metA ou B gal::Tn5 malA strA xyl mtl ampA1 phoR51 zib::Tn10 |
| PC0479 | F⁻ | thr leu thi pyrF thy ilvA his argG lacY supE tsx tonA rpsL |
| 2671 | F⁻ | thr leu thi pyrF thy ilvA his argG lacY supE tsx tonA rpsL gal::Tn5 ampA1 phoR51 phoU zib::Tn10 |
| 207 | Hfr | met lky207 |
| 2851 | Hfr | met ampA1 phoR51 phoU zib::Tn10 lky207 |
| 3181 | Hfr | met ampA1 phoR51 phoU zib::Tn10 hyp3042 lky207 |
| 3233 | Hfr | met ampA1 phoR51 phoU zib::Tn10 gal::Tn5 PBR322 |
| 3269 | Hfr | met ampA1 phoR51 phoU zib::Tn0 lky207 PBR322 |
| 3193 | Hfr | met ampA1 phoR51 phoU zib::Tn10 gal::Tn5 PBR322 hyp3031 |

```
3661   Hfr   met ampA1 phoR51 phoU zib::Tn10 lky207 PBR322
             hyp3031
3190   Hfr   met ampA1 phoR51 phoU zib::Tn10 gal::Tn5 PBR322
             hyp3042
3272   Hfr   met ampA1 phoR51 phoU zib::Tn10 lky207 PBR322
             hyp3042
3679   Hfr   met ampA1 phoR51 phoU zib::Tn10 gal::Tn5 PJC2431
3691   Hfr   met ampA1 phoR51 phoU zib::Tn10 gal::Tn5 hyp3032
             PJC2431
3688   Hfr   met ampA1 phoR51 phoU zib::Tn10 gal::Tn5 hyp3033
             PJC2431
3680   Hfr   met ampA1 phoR51 phoU zib::Tn10 gal::Tn5 hyp3034
             PJC2431
3687   Hfr   met ampA1 phoR51 phoU zib::Tn10 gal::Tn5 hyp3039
             PJC2431
3683   Hfr   met ampA1 phoR51 phoU zib::Tn10 gal::Tn5 hyp3041
             PJC2431
3693   Hfr   met ampA1 phoR51 phoU zib::Tn10 gal::Tn5 hyp3042
             PJC2431
PEC590       sans exigence plasmide col K
PRC594       nalA plasmide col E₂-P9
PRC599       nalA plasmide col Ib-P9
PRC628       sans exigence plasmide col A-CA31
B232103      sans exigence plasmide col D-CA23
B232123      gyrA plasmide col N-284
```

La souche parentale 2590 a été déposée à la Collection Nationale de Culture des Micro-organismes

---

0 267 110

sous le n° I-605

avec indication des propriétés suivantes :

Les mutations phoU, phoR51, ampA$_1$ ainsi que l'insertion du transposon Tn10 à la minute 81 (zib::Tn10) ont été transférées dans une souche d'E. coli (HfrC, proC, metB, gal::Tn5) par tranduction à l'aide du bactériophae P1 selon la technique décrite dans (Miller J. (1977) Experiments in Molecular Genetics, Cold Spring Harbor Laboratory, Cold Spring Harbor N.Y.)

Cette souche synthétise peu de phosphatase alcaline mais de manière constitutive (mutations phoU, phoR51).

Elle est résistante à 10 µg/ml d'ampicilline (mutation ampA$_1$, mutation de type "promoteur up" du gène de structure ampC de la β-lactamase chromosomique) et à 50 µg/ml de kanamycine (présence du transposon Tn5 qui porte le gène de structure de l'aminoglycoside 3'o phosphotransférase APH3').

2. Milieux de culture.

On a utilisé les milieux suivants :

Milieux riches.

Milieu BL : extrait de levure (5g/l) bactotryptone (10g/l) et chlorure de sodium (5g/l) pH 7,3 : (Miller J., (1972), Experiments in Molecular Genetics, Cold Spring Harbor Laboratory).

BL 83 : milieu BL pH : 8,3.

Milieux synthétiques.

Milieu 63 : $KH_2 PO_4$ (0,1 M) ; $(NH_4)2 SO_4$ (15 mM) ; $Mg SO_4 7H_2O$ (4 mM) ; $FeSO_4 7H_2O$ (9µM) ; KOH (0,2 mM) pH : 7,2 (Pardel, Jacob et Monod, (1959), J. Mol. Biol. 1 165-171).

Milieu Tris : milieu carencé en phosphate : Tris (0, 12 M) ; NaCl (0,09 M) ; KCl (0,04 M) ; $NH_4$ Cl (0,021 M) ; $CaCl_2$ (O,4 mM) ; $MgCl_2$ (1 mM) ; Fe $Cl_3$ (7 mM) ; $Na_2 SO_4$ (0,025 mM) pH 8,3 (Worcel A., Burg E (1974) J. Mol. Biol. 82 91-105).

Milieu TBL 8,3 : 100 ml de milieu Tris + 8 ml de milieu BL 8,3.

Les milieux solides contiennent 12g/l d'agar. Les plus utilisés sont les suivants : milieu riche BL et milieu synthétique M63.

Les facteurs de croissance nécessaires sont ajoutés aux milieux synthétiques, aux concentrations suivantes : méthionine 0,4 g/l, proline, arginine, histidine, thréonine et leucine 0,1g/l,adénine 0,03g/l, thymine 0,05g/l, vitamine B1 0,5 mg/l. Les sucres utilisés comme source de carbone sont ajoutés aux concentrations suivantes : glucose 4g/l ; succinate 6,5g/l ; ribose 4g/l ; mannitol 4g/l ; xylose 4g/l.

3. Plasmides utilisés.

Il s'agit des plasmides suivants :

PBR322 : bla+

PJC2431 : phoA+ bla+ (JC. Lazzaroni, D. Atlan, R. Portalier J. Bact. (1985) 164 1376-1380).

4. Cultures bactériennes.

Les précultures sont effectuées en milieu riche BL à 37°C et sous agitation mécanique. La croissance bactérienne est estimée par l'estimation de la turbidité des suspensions, mesurée à 600 nm à l'aide d'un spectrophotomètre JOUAN (0,4 unités de densité optique correspondant à une concentration cellulaire de $3.10^8$ bactéries par ml, soit 0,2 mg de poids sec bactérien par ml).

5. Extraction enzymatique.

Le contenu cellulaire des bactéries est libéré après éclatement des cellules sous l'action d'une pression mécanique élevée (20 000 psl à +4°C) à l'aide d'une presse de FRENCH. Les débris membranaires et les cellules non éclatées sont éliminées par centrifugation pendant 10 mn à 10 000 g.

6. Détermination des activités enzymatiques.

Les activités enzymatiques sont déterminées par les méthodes décrites par :

-Garen et Levinthal, (1960), Biochem. Biophys. Acta, 38 470-483 pour la phosphatase alcaline.

-Miller, (1972), Experiments in Molecular Genetics. Cold Spring Harbor Laboratory, Cold Spring Harbor New-York pour la β-galactosidase.

-Sykes et Nordström (1972), Antim. Agents chemother. 1, 94-99 pour la β-lactamase.

-Goldman et Northrop, (1976), Biochem. Biophys. Acta, 69, 230-236 pour l'aminoglycoside 3' phosphotransférase (APH3').

-Lazdunski A, Murgier M. et Lazdunski C, (1975), Eur. J. Biochem. 60 349-355 pour l'aminoendopeptidase.

Une unité d'activité enzymatique (U) correspond à la quantité d'enzyme qui transforme :

-une nanomole de substrat par minute, dans le cas de la β-galactosidase, de l'APH3' et de l'aminoendopeptidase (1U = 16,67 pkat)

-une micromole de substrat par minute, dans le cas de la β-lactamase (1U = 16,67 nkat).

Par ailleurs, une détection colorée de l'activité de la phosphatase alcaline est effectuée au niveau des colonies bactériennes par un test qualitatif in situ : un mélange d'un volume égal d'α-naphtyl phosphate [(8 mM) en Tris HCl (1M) à pH = 8,0] et d'o-dianisidine tétrazotée (20 mM dans l'eau) est versé sur les colonies qui se sont développées sur milieu solide. L'α-naphtol libéré après l'hydrolyse de l'α-naphtyl-phosphate par la phosphatase alcaline, forme avec l'o-dianizidine tétrazotée, un précipité brun (Miller, 1972).

## 7. Résistance aux agents anti-bactériens.

### - Résistance aux colicines.

La production des colicines $E_2$ ; A ; K ; Ib ; D ; et N est réalisée à l'aide des souches colicinogéniques PRC 594, PRC 628, PRC 590, PRC 599, B232102, B232123 respectivement, selon la méthode décrite par Spudich et al, (1970), J. Mol. Biol. 53 49-67.

$10^9$ bactéries de la souche étudiée sont étalées sur milieu riche solide. On ajoute sur ce tapis une goutte (0,05 ml) de différentes dilutions de la solution concentrée de colicine à tester et on incube les boîtes de Pétri à 37°C pendant 18 heures. Les bactéries qui ne se développent pas en présence d'une dilution donnée de colicine sont dites sensibles à cette concentration.

### - Sensibilité aux bactériophages.

La résistance ou la sensibilité des souches aux bactériophages est estimée de façon qualitative par la méthode dite du "cross-streaking", qui consiste à déposer sur un milieu riche solide BL une goutte d'un lysat de phages (environ $10^9$ phages/ml) sous la forme d'une ligne, et une goutte de la souche à tester (environ $10^9$ bactéries/ml) sous forme d'une autre ligne qui croise la première de façon perpendiculaire. Le mutant est dit résistant au phage si la croissance n'est pas altérée après la rencontre du lysat, ou sensible dans le cas contraire.

### -Détermination des concentrations minimales inhibitrices.

300 cellules bactériennes environ sont déposées sur des boîtes de milieu riche BL contenant un antibiotique à une concentration donnée. Après 18 heures d'incubation à 37°C, la plus faible concentration d'antibiotique inhibant totalement la croissance bactérienne (CMI) est identifiée.

## 8. Transfert de matériel génétique.

La préparation de lysats de bactériophages P1, les transductions généralisées par P1 ainsi que les croisements non interrompus ont été effectués selon les protocoles décrits (Miller, 1972...)

## 9. Transformations des cellules bactériennes par l'ADN plasmidique.

Le traitement des cellules d'E. coli par du chlorure de calcium "perméabilise" l'enveloppe cellulaire et permet la pénétration dans le cytoplasme de molécules d'ADN plasmidique.

Les plasmides ainsi introduits demeurent dans les cellules hôtes, sont doués d'autoréplication et sont transmis à la descendance.

Les cellules d'E. coli ont été transformées par le plasmide PBR322 et le plasmide PJC2431 selon une méthode dérivée de celle décrite par Ledeberg et Cohen, (1974), J. bactériol. 119 1072-1074.

## 10. Sélection des mutants hyp.

Plusieurs milieux (milieu BL, milieu BL additionné de phosphate $10^{-1}$ et $10^{-2}$ M et milieu synthétique M63) ont été testés pour évaluer les concentrations minimales inhibitrices de la souche parentale vis-à-vis de l'ampicilline et de la kanamycine. Une forte concentration en phosphate augmente profondément la valeur de la concentration minimale inhibitrice de la kanamycine vis-à-vis de la souche parentale, aussi seul le milieu BL a été retenu.

Des milieux de croissance à base de milieu BL ont été réalisés, contenant soit de l'ampicilline seule aux concentrations de 50, 100, ou 200 µg/ml, soit de la kanamycine seule aux concentrations de 100, 200 ou 300 µg/ml, soit toutes les combinaisons possibles des ces antibiotiques utilisés à l'une quelconque des concentrations précitées.

Les mutants hyp ont été obtenus sur des milieux contenant soit les deux antibiotiques (ampicilline : 100 µg/ml et kanamycine : 100, 200 ou 300 µg/ml en utilisant un inoculum de $10^8$ bactéries), soit sur un milieu contenant 100 µg/ml d'ampicilline seulement avec un inoculum de $10^7$ bactéries.

Une concentration plus faible d'ampicilline en présence ou en absence de kanamycine permet le développement d'un tapis bactérien et inversement, une concentration plus forte empêche toute croissance de la souche parentale.

La durée de la culture est de 18 heures.

On recherche ensuite parmi les mutants antibiorésistants ainsi mis en évidence, ceux qui révèlent une activité enzymatique spécifique supplémentaire accrue. Cette recherche, par la méthode classique (screening), revèle de 2 à 5 % de mutants qui sont les mutants hyp.

Le taux d'obtention des mutants par rapport à la souche parentale est de $10^{-8}$.

11. Analyse enzymatique des mutants obtenus.

Activités enzymatiques spécifiques relatives

| Souches | phosphatase alcaline | β-lactamase | APH3' | aminoendo-peptidase | β-galactosidase |
|---------|---------------------|-------------|-------|---------------------|-----------------|
| souche parentale 2590 | 1 | 1 | 1 | 1 | 1 |
| mutants. hyp | | | | | |
| 3031 | 2,2 | 1,7 | 1,2 | 1,3 | 1,4 |
| 3032 | 2,1 | 1,7 | 2,4 | 1,5 | 1,8 |
| 3033 | 2 | 1,9 | 1 | 1,5 | 1,9 |
| 3034 | 2,1 | 1,3 | 3,3 | 1,3 | 1,3 |
| 3035 | 1 | 3,3 | 3,5 | 1 | 1 |
| 3036 | 4,8 | 2 | 1,3 | 1 | 1,5 |
| 3037 | 1 | 2,8 | 2,4 | 1 | 1,3 |
| 3038 | 2,2 | 1,3 | 3,8 | 1 | 1 |
| 3039 | 2,6 | 1,6 | 3 | 1,3 | 1 |
| 3040 | 2,3 | 1,5 | 1,6 | 1,2 | 1,2 |
| 3041 | 2,2 | 2,1 | 3,6 | 1,5 | 1,9 |
| 3042 | 2,7 | 1,8 | 3,8 | 1,5 | 1,4 |
| 3043 | 0,7 | 2,3 | 2,4 | 1 | 1,3 |
| 3044 | 1,6 | 1,5 | 1,2 | 1,6 | 1,2 |

Tableau 1 : Accroissement des activités alcaline, β-lactamase et PAH3' chez les mutants hyp.

Les activités spécifiques de la souche parentale sont prises comme base 1. Les résultats portés dans ce tableau sont reproductibles (3 expériences indépendantes).

Les mutants hyp présentent des profils assez variés. Certaines souches sont caractérisées par l'exaltation simultanée des trois enzymes choisies comme sondes, d'autres par l'exaltation de deux activités seulment. Le taux d'accroissement maximum est de 5.

On not également dans certains cas, une faible augmentation des activités de la β-galactosidase et de l'aminopeptidase.

12. Analyse phénotypique des mutants hyp.

Plusieurs sous-classes de mutants hyp ont été distinguées en fonction d'une part de leur aptitude ou de leur incapacité, à utiliser différents sucres comme sources de carbone et d'autre part sur la base de leur sensibilité ou de leur résistance accrues à diverses drogues inhibitrices de la croissance bactérienne, aux colicines ou aux bactériophages.

| | souche | Classe A | Classe B | Classe C | Classe D | Classe E | Classe F |
|---|---|---|---|---|---|---|---|
| | parentale | 3034 | | | | | |
| milieu de | 2590 | 3038 | | | | 3032 | 3031 |
| croissance | | 3039 | 3041 | | | 3033 | 3036 |
| | | 3040 | 3042 | 3044 | 3035 | 3043 | 3037 |
| AC | + | - | - | + | + | + | + |
| OA | + | - | - | - | + | + | + |
| rif | - | - | - | + | - | - | + |
| ribose | + | - | + | - | + | + | + |
| succinate | +/- | +/- | +/- | +/- | +/- | + | + |

AC : acide cholique 2,5%       + : croissance

OA : orange acridine 0,5 mg/ml      - : non croissance

rif : rifampicine 20 µg/ml      +/- : croissance faible

## Tableau 2 : Analyse des propriétés phénotypiques des mutants hyp.

Les classes A, C, et D regroupent des mutants dont le taux de synthèse de la $\beta$-galactosidase reste inchangé.

Les classes E et F regroupent des mutants présentant ou non une augmentation du taux de synthèse de la $\beta$-galactosidase.

La classe B est constituée de mutants dont le taux de synthèse de la $\beta$-galactosidase est augmenté.

### Etude de la sensibilité aux colicines.

Le tableau suivant indique les concentrations maximales de colicines permettant la croissance des bactéries.

| colicines | Souche parentale : 2590<br>3033 3035 3037 3043 | 3031 | 3044 | 3034<br>3040 | 3036 | 3041 | 3039 | 3042 | 3038 | 3032 |
|---|---|---|---|---|---|---|---|---|---|---|
| A | $10^{-3}$ | - | - | - | $10^{0}$ | $10^{-2}$ | - | $10^{-2}$ | $10^{-2}$ | $10^{0}$ |
| K | $10^{-2}$ | $10^{-3}$ | - | - | $10^{-1}$ | - | $10^{-2}$ | $10^{0}$ | - | $10^{0}$ |
| Ib | $10^{-2}$ | - | $10^{0}$ | - | - | - | - | - | - | $10^{0}$ |
| D | $10^{-3}$ | - | - | - | - | $10^{-2}$ | - | - | $10^{-4}$ | - |
| $E_2$ | $10^{-3}$ | - | - | - | - | - | - | - | - | $10^{-2}$ |
| N | $10^{-2}$ | - | - | $10^{-1}$ | - | - | $10^{-1}$ | $10^{-1}$ | $10^{-1}$ | $10^{-1}$ |

$10^{0}$ : mutant résistant à la colicine pure

- : même sensibilité que la souche parentale

La plupart des mutants <u>hyp</u> manifestent une résistance accrue à une ou plusieurs colicines.

Tableau 3 : Sensibilité des mutants hyp vis-à-vis des colicines.

Sensibilité aux bactériophages.
La sensibilité aux bactériophages a été testée par cross-streaking.

| souches / phages | souche parenterale 2590 3032-3033-3043 | 3034 3038 | 3041 | 3044 | 3035 | 3039 | 3036 3037 | 3031-3040 3042 |
|---|---|---|---|---|---|---|---|---|
| T3 | R | R | S | S | S | S | S | S |
| T4 | R | R | R | S | S | S | S | S |
| T7 | R | R | R | R | R | R | S | S |
| λvir | R | R | R | R | S | R | S | R |
| C21 | R | R | R | R | R | S | R | S |
| Br10 | S | R | R | S | S | S | S | S |
| Br2 | S | R | R | S | S | S | S | S |

R : résistant

S : sensible

Tableau 4 : Sensibilité des mutants hyp vis-à-vis des bactério-phages.

La plupart des mutants hyp sont plus sensibles aux bactério-phages que la souche parentale.

Détermination des concentrations minimales inhibitrices (CMI) vis-à-vis de l'ampicilline et de la kanamycine pour différents mutants hyp.

Les CMI ont été déterminées sur milieu riche solide BL après 18 heures de croissance à 37°C.

| Souches / CMI empg/ml | parent | 3031-3032-3033 3036-3037-3043 3044 | 3034-3038 3041 | 3035 3040 | 3039 3042 |
|---|---|---|---|---|---|
| | 2590 | | | | |
| ampicilline | 40-50 | 100-140 | 50-70 | 110-140 | 80-100 |
| Kanamycine | 70-80 | 150-200 | 450-550 | 500 | 1000 |

**Tableau 5 : Détermination des concentrations minimales inhibitrices (CMI) des mutants hyp vis-à-vis de l'ampicilline et de la kanamycine.**

Les CMI obtenues pour l'ampicilline sont en rapport direct avec les taux de synthèse de $\beta$-lactamase estimés pour chaque mutant.

13. Localisation génétique de la mutations hyp.

Afin de mettre en évidence le transfert d'une mutation chez une souche réceptrice, il faut que cette dernière puisse synthétiser les trois enzymes retenues ($\beta$-lactamase, phosphatase alcaline et APH3') aux mêmes niveaux de synthèse que la souche parentale utilisée pour l'isolement des mutants. Il a donc été nécessaire d'introduire les marqueurs génétiques ampA1, phoU, phoR51, gal::Tn5 chez toutes les souches chez lesquelles les mutations devaient s'exprimer.

Pour pouvoir localiser par conjugaison les mutations hyp sur le chromosome d'E. coli, on a construit 3 souches F- portant, d'une part, les marqueurs génétiques mentionnés ci-dessus et, d'autre part, différentes combinaisons de marqueurs génétiques réparties régulièrement le long du chromosome bactérien.

Des croisements non-interrompus ont été réalisés successivement entre les souches donatrices Hfr (hyp) et les souches F- réceptrices 2671 et 2902...

L'analyse de la ségrégation des marqueurs non sélectionnés chez les recombinants Hyp-, ou prototrophes a conduit à situer le locus hyp dans la région malA mtl.

Après transduction de la souche réceptrice 2902 à l'aide d'un lysat de bactériophages P1 réalisé sur des mutants hyp, des transductants Ileu+, Xyl+, Mtl+ ont été sélectionnés et analysés. Aucune cotransduction n'a été mise en évidence entre la mutation hyp et les marqueurs ilvA et xyl. Par contre, la mutation hyp est cotransductible à une fréquence de 25% avec le marqueur mtl. La mutation hyp est donc localisée vers la minute 81 du chromosome d'E. coli.

Des expériences analogues ont été réalisées en utilisant quatorze mutants hyp indépendants. Toutes les mutations hyp caractérisées ont été localisées vers la minute 81.

14 - Etude de l'expression de la mutation hyp dans différents contextes génétiques.

La souche parentale 2590 à partir de laquelle les différentes mutations hyp ont été isolées a été construite à l'origine de telle sorte que la production des enzymes choisies comme marqueurs (phosphatase alcaline, $\beta$-lactamase et APH3') soit faible, ceci afin de faciliter l'identification des mutants.

Il était donc intéressant d'évaluer l'expression des mutations hyp dans d'autres contextes génétiques permettant une synthèse plus importante des différentes enzymes sondes.

L'influence de la présence de la mutation exc876 permettant la libération de certaines enzymes périplasmiques (phosphatase alcaline, $\beta$-lactamase) dans le milieu extracellulaire a été également étudiée.

Influence de la mutation exc876 sur l'expression de la mutation hyp.

La mutation exc876 a été introduite par cotransduction avec le marqueur gal dans le génome de différents mutants hyp : voir Fognini-Lefebvre N., Portalier R., 1984, Isolation and preliminary characterization of $\beta$-lactamase excretory mutants of Escherichiacoli K-12, FEMS Microbiology Letters 21, 323-328, et Lazzaroni J-C, Portalier R., 1981, Genetic and biochemical characterization of periplasmic-leaky mutants of Escherichia coli K-12, J. Bacteriol., 145, 1351-1358.

Les résultats de l'analyse des différentes souches, parentale et mutantes, sont résumés dans le tableau suivant :

| Souches | Production spécifique totale en U/mg de PSB | | |
|---|---|---|---|
| | phosphatase alcaline | aminoendo-peptidase | β-galactosi-dase |
| 2590  hyp+,exc+) | 60 | 40 | 2300 |
| 2851  (hyp+exc876) | 40 | 30 | 2000 |
| 3042  (hyp3042 exc+) | 100 | 60 | 2300 |
| 3181  (hyp3042exc876 | 180 | 30 | 2500 |

## Tableau 6 : Influence de la mutation exc876 sur l'expression de la mutation hyp3042.

La présence simultanée des mutations hyp3042 et exc876 chez la souche 3181 augmente significativement l'activité de la phosphatase alcaline par rapport aux activités observées chez les souches 3042 (hyp-) et 2851 (exc-). Ce résultat tend à prouver que l'expression de la mutation hyp3042 n'est pas maximale dans le contexte génétique des mutants d'origine.

Influence de la présence du plasmide PBR322 (bla+) sur l'expression de la mutation hyp.
Trois classes de souches ont été construites et analysées. Il s'agit
- de souches exc+ (non excrétrices) portant ou non une mutation hyp
- de souches exc+ transformées par le plasmide PBR322 (PBR) et portant ou non une mutation hyp
- de souches exc- (excrétrices) transformées par le plasmide PBR322 et portant ou non une mutation hyp.
La présence du plasmide PBR322 permet une synthèse beaucoup plus importante de β-lactamase, enzyme périplasmique excrétée à des taux élevés dans le milieu de culture par les mutants exc.
Les résultats obtenus sont présentés dans le tableau suivant (ils correspondent aux valeurs moyennes obtenues lors de trois expériences).

0 267 110

Production spécifique dans le surnageant : activité enzymatique extracellulaire (U)/mg de poids sec bactérien. (Les nombres entre parenthèses représentent les pourcentages d'enzyme excrétée).

| Activités enzymatiques / génotypes | production spécifique totale U/mg de poids sec bactérien | | | production spécifique dans le surnageant de culture U/mg PSB | | β-galactosidase (% de lyse) |
|---|---|---|---|---|---|---|
| | β-lactamase | phosphatase alcaline | aminoendo-peptidase | β-lactamase | phosphatase alcaline | |
| 2590 : hyp$^+$ exc$^+$     a | 2,8 10$^{-3}$ | 58 | 45 | | | |
| 3233 : hyp$^+$ exc$^+$ PBR   b | 4,8 | 50 | 33 | | | |
| 3269 : hyp$^+$ exc876 PBR   c | 9,9 | 53 | 29 | 8,5 (86) | 38 (72) | (1,5) |
| 3031 : hyp3031exc+    d | 4,9 10$^{-3}$ | 130 | 60 | | | |
| 3193 : hyp3031exc$^+$ PBR e | 7,6 | 130 | | | | |
| 3661 : hyp3031 exc876PBR f | 17,5 | 160 | | 14,8 (85) | 125 (78) | (1,8) |
| 3042 : hyp3042 exc$^+$    g | 5,2 10$^{-3}$ | 130 | 68 | | | |
| 3190 : hyp3042 exc$^+$ PBR h | 9,1 | 110 | 45 | | | |
| 3272 : hyp3042 exc876 PBR i | 12,6 | 205 | 40 | 10 (79) | 160 (78) | (1,6) |

Tableau 7 : Influence de la présence du plasmide PBR322 sur l'expression des mutations hyp3031 et hyp3042.

Production spécifique totale : production spécifique intra- plus extracellulaire.

L'activité β-lactamase est considérablement augmentée après transformation des souches par le plasmide PBR322 (facteur × 1700) (comparer les souches a et b, d et e, ou g et h). Malgré cette exaltation, les mutations hyp3031 et hyp3042 s'expriment parfaitement dans ces conditions de synthèse élevée (comparer les souches b, e et h).

La présence simultanée des mutations exc876 et hyp chez une même souche est associée à des taux de synthèse de β-lactamase et de phosphatase alcaline supérieurs à ceux mesurès chez des souches ne portant que l'une de ces deux mutations (comparer les activités des souches f et i, d'une part et celles des souches c, e et h d'autre part).

Dans les surnageants de culture, les quantités d'enzyme excrétée par les doubles mutants hyp-exc- sont augmentées d'un facteur 2 (pour la β-lactamase souche f) ou 4 (pour la phosphatase alcaline souche i) et ceci dans des conditions où la lyse bactérienne est inférieure à 2%.

Les résultats présentés ci-dessus démontrent l'intérêt des mutants hyp pour accroître les rendements de production de protéines d'intérêt biologique, chez E. coli, notamment dans le cas où ces protéines sont excrétées.

Influence de la présence du plasmide PJC 2431 (phoA+) sur l'expression de la mutation hyp.

Le plasmide pJc 2431 (JC.Lazzaroni, D. Atlan, R. Portalier (1985) J. Bacteriol. 164 1376-1380) portant les gènes de structure de la phosphatase alcaline (phoA+) et de la β-lactamase (bla) a été introduit par transformation dans les différentes souches hyp.

La phosphatase alcaline appartient au régulon phosphate. Chez la souche sauvage la synthèse de l'enzyme est réprimée en présence d'un excès de phosphate inorganique dans le milieu de culture des bactéries. L'introduction dans le génome d'E Coli des mutations phoR51 et phoU (souche 2590 et dérivés hyp) rend la synthèse de la phosphatase alcaline faiblement "constitutive", c'est-à-dire effective même en présence d'un excès de phosphate dans le milieu de culture.

La phosphatase alcaline a été dosée après croissance des bactéries dans deux types de milieu : à faible (milieu TBL) ou à forte (milieu BL) concentration en phosphate. Le pH de ces deux milieux a été ajusté à 8,3.

|  | MILIEU BL 8,3 | | | | | MILIEU TBL 8,3 | | | | |
|  | phosphatase alcaline | | lactamase | | galactosidase | phosphatase alcaline | | lactamase | | galactosidase |
|  | PST | % excrétion | PST | % excrétion | % lyse | PST | % excrétion | PST | % excrétion | % lyse |
|---|---|---|---|---|---|---|---|---|---|---|
| 2590 hyo+ | 59 | 2 | $2{,}9\ 10^{-3}$ | – | 0,25 | | | | | |
| hyp 3042 | 160 | 4 | $5{,}2\ 10^{-3}$ | – | 4 | | | | | |
| 3679 hyp+ pJC2431 | 1068 | 1,9 | 0,02 | 35 | 1 | 1006 | 5,3 | 0,47 | 12 | 1,9 |
| 3691 hyp 3032 pJC2431 | 2229 | 4,2 | 0,06 | 20 | 1 | 3543 | 12 | | | 3 |
| 3688 hyp3033 pJC2431 | 1640 | 2,8 | 0,02 | 26 | 1 | 4958 | 7 | 0,42 | 15 | 2,4 |
| 3680 hyp 3034 pJC2431 | 1559 | 26 | 0,15 | 63 | 5 | 2438 | 39 | 0,9 | 56 | 8,3 |
| 3687 kyp3039 pJC2431 | 1619 | 21 | 0,1 | 73 | 3 | 2646 | 22 | 0,48 | 5 | 7,8 |
| 3683 kyp3041 pJC2431 | 2497 | 28,7 | 0,17 | 54 | 3 | 3924 | 25 | 0,9 | 29 | 8 |
| 3693 kyp3042 pJC2431 | 1812 | 14,7 | 0,08 | 57 | 4 | 4687 | 24 | 1,2 | 2 | 3,9 |

Tableau 8

PST : production spécifique totale en U/mg de poids sec bactérien.

EMI PA = 25 FR = 1 HE = 15 WI = 160 TI = TAB

L'augmentation de la production spécifique totale de phosphatase alcaline due à la présence des mutations hyp est plus importante dans le milieu TBL 8,3. Dans ces conditions de croissance, en présence du plasmide pJC 2431 et de la mutation hyp 3033, la production de phosphatase alcaline est augmentée d'un facteur 5. Les résultats présentés dans le tableau 8 montrent que l'effet d'exaltation de la synthèse de la phosphatase alcaline dû à la présence des mutations hyp s'exerce bien même lorsque les cellules produisent des quantités élevées d'enzyme. En effet, les souches transformées par le plasmide pJC 2431 produisent 20 fois plus de phosphatase alcaline que les souches haploïdes. Chez certains mutants hyp on observe une excrétion de la phosphatase alcaline dans le milieu de culture. Les pourcentages d'excrétion obtenus sont comparables à ceux décrits par JC. Lazzaroni et R. Portalier. (JC. Lazzaroni, D. Atlan, R. Portalier J. Bacteriol. 164 1376-1880). Il faut cependant noter que dans le système étudié par ces auteurs, les souches transformées par le plasmide pJC 2431 portaient une mutation phoS ou pstB et qu'en présence d'une seule mutation phoR ou phoU les souches transformées n'excrétaient pas significativement la phosphatase alcaline.

De même que pour la phosphatase alcaline, la β-lactamase est synthétisée en plus grande quantité durant la croissance en milieu TBL. Par contre, l'effet d'exaltation des mutations hyp et les taux d'excrétion sont plus élevés lorsque les bactéries se développent en BL.

### 15. Application des souches hyp à la production de protéines.

Les souches hyp, faciles à cultiver, sont utilisables pour obtenir des préparations purifées de protéines désirables qui sont excrétées dans le surnageant de culture qui ne se trouve que très faiblement contaminé par d'autres espèces de protéines.

A cette fin, on peut avantageusement utiliser l'association entre la mutation hyp et un mode d'excrétion spécifique, par exemple par présence dans la bactérie du plasmide p5C2431.

On peut également associer la mutation hyp et un mode d'excrétion pléïotrope, par exemple par présence simultanée de la mutation ex 876.

Bien entendu, l'invention n'est pas limitée à la production des seuls enzymes décrits et on comprend qu'elle s'étend à l'application à la production sous forme purifiée de toute autre protéine produite et/ou excrétée en quantité accrue par la mutation hyp, éventuellement associée à une mutation hyperexcrétrice. En particulier, on peut cloner un gène de structure d'une protéine intéressante sur un plasmide et procéder à la fusion de ce gène avec le gène de structure d'une protéine (notamment phosphatase alcaline et/ou β-lactamase), créant ainsi dans la souche hyp un gène exprimant une protéine hybride dont la mutation hyp, associée de préférence, à l'une des mutations d'excrétion précitées, assurera une amplification de la production.

## Revendications

1. Nouvelles souches d'Escherichia coli hyperproductrices ou simultanément hyperproductrices et hyperexportatrices de protéines exportées, caractérisées en ce qu'elles comportent une mutation, dite hyp, localisée dans la région mAIA mtl vers la minute 81, ladite mutation étant cotransductible à une fréquence d'environ 20 à 25% par le bactériophage P1 avec le marqueur mtl, ladite mutation étant responsable d'une hyperproduction (et/ou d'une hyperexportation) d'au moins deux protéines d'origine périplasmique présentes, mais en quantité au moins 2 à 5 fois plus faible, dans la souche parentale.

2. Nouvelles souches selon la revendication 1 et déposées auprès de la Collection Nationale de Cultures de microorganismes et respectivement accessibles sous les no
   I-601, I-602, I-603, I-604.

3. Nouvelles souches selon l'une des revendications 1 et 2 dont la mutation, dite hyp, est responsable d'une hyperproduction de la phosphatase alcaline et/ou de la β-lactamase et/ou de l'aminoglycoside 3' phosphotransférase (APH 3').

4. Nouvelles souches selon l'une quelconque des revendications 1 à 3, caractérisées en ce qu'elles comportent également une mutation permettant l'excrétion et augmentant la production de protéines périplasmiques, notamment la mutation exc876.

5. Procédé d'obtention des souches selon l'une quelconque des revendications 1 à 4, caractérisé en ce que l'on choisit ou construit une souche parentale d'Escherichia coli synthétisant, à un niveau au moins décelable, la ou les protéines dont on désire obtenir l'exaltation de la production et/ou de l'excrétion, protéines parmi lesquelles figure une enzyme susceptible de détruire un agent anti-bactérien, tel que, de préférence, un antibiotique, certaines au moins desdites protéines ou enzymes n'appartenant pas au même régulon, et présente dans le milieu intra-cellulaire à une concentration au moins décelable mais loin de la saturation, que l'on cultive la souche parentale et que l'on sélectionne la ou les bactéries présentant une synthèse accrue de ladite enzyme et une résistance accrue auxdits agents anti-bactériens, le taux d'obtention des mutants étant de l'ordre de 10

6. Procédé selon la revendication 5, caractérisé en ce que l'on cultive une souche parentale, notamment E. coli K-12, notamment sur milieu BL, et en présence d'ampicilline à 100 µg/ml avec ou sans kanamycine (100, 200 ou 300 g/ml).

7. Application d'une souche selon l'une quelconque des revendications 1 à 4, à la production accrue de protéines, caractérisée en ce qu'on cultive la souche dans un milieu de culture liquide et que l'on sépare le liquide d'avec les bactéries.

8. Application selon la revendication 7 à la production de phosphatase alcaline et/ou de β-lactamase et/ou d'amino-glycoside 3' phospho-transférase, ou d'une protéine hybride incorporant l'un desdits enzymes.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 149 936 (INSTITUT MERIEUX) * Résumé; page 12, lignes 3-31; revendications * & FR-A-8 320 022 (Cat. D) --- | 1 | C 12 N  15/00 C 12 N   1/20 // C 12 P  21/02 (C 12 N   1/20 C 12 R   1:19 ) |
| A | WO-A-8 604 089 (INSTITUT MERIEUX) * Page 2, ligne 21 - page 4, ligne 36; tableau 2; revendications * & FR-A-8 500 087 (Cat. D) --- | 1 | |
| A | BIOLOGICAL ABSTRACTS, vol. 81, no. 6, 1986, résumé no. 52487, Philadelphia, PA, US; J.C. LAZZARONI et al.: "Excretion of alkaline phosphatase by Escherichia coli K-12 pho constitutive mutants transformed with plasmids carrying the alkaline phosphatase structural gene", & J. BACTERIOL., vol. 164, no. 3, 1985, pages 1376-1380 * Résumé * ----- | 1 | |

**DOMAINES TECHNIQUES RECHERCHES (Int. Cl.4)**

C 12 N

Le présent rapport a été établi pour toutes les revendications

| Lieu de la recherche | Date d'achèvement de la recherche | Examinateur |
|---|---|---|
| LA HAYE | 09-12-1987 | YEATS S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)